(19) Europäisches Patentamt

European Patent Office

Office européen des brevets

(11) Publication number: **0 261 275 B1**

# (12) EUROPEAN PATENT SPECIFICATION

(45) Date of publication of patent specification: **04.12.91**   (51) Int. Cl.5: **G01N 27/12**

(21) Application number: **86113291.8**

(22) Date of filing: **26.09.86**

(54) A hydrogen gas detecting element and method of producing same.

(43) Date of publication of application:
**30.03.88 Bulletin 88/13**

(45) Publication of the grant of the patent:
**04.12.91 Bulletin 91/49**

(84) Designated Contracting States:
**BE DE FR IT NL**

(56) References cited:
**EP-A- 0 115 183**
**EP-A- 0 147 213**
**US-A- 4 583 070**

**PATENT ABSTRACTS OF JAPAN, vol. 10, no. 111 (P-451)[2168], 25th April 1986; & JP-A-60 243 548**

**PATENT ABSTRACTS OF JAPAN, vol. 9, no. 224 (P-387)[1947], 10th September 1985; & JP-A-60 82 954**

(73) Proprietor: **NOHMI BOSAI LTD.**
**7-3, Kudan Minami 4-chome, Chiyoda-ku Tokyo 102(JP)**

(72) Inventor: **Okayama, Yoshiaki Nohmi Bosai Kogyo Co. Ltd.**
**7-3, Kudan Minami 4-chome Chiyoda-ku Tokyo(JP)**

(74) Representative: **Dr. Elisabeth Jung Dr. Jürgen Schirdewahn Dipl.-Ing. Claus Gernhardt P.O. Box 40 14 68 Clemensstrasse 30 W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Abstract

A hydrogen gas detecting element is produced by treating an element body in a silane gas atmosphere having a silane content of 500 to 5,000 ppm, said element comprising Pd or Pd and Pt, or Pd, Pt and Sb dispersed on $SnO_2$, Pd/Sn being 0.1 to 8 mol%, Pt/Sn being 0 to 8 mol% and Sb/Sn being 0 to 8 mol%.

The element shows high selectivity and short response time in detecting hydrogen gas.

Field of the Invention

The present invention relates to a hydrogen gas detecting element and a method of producing such an element.

Prior Art

As a hydrogen gas detecting element using a metal oxide, a hydrogen-sensitive sensor is known, in which a hydrogen molecule-selective permeable film, e.g., an inflammable thin film is formed on a gas-sensitive element. In such sensors, however, the hydrogen gas must pass through the thin film filter and thus some time is needed until the hydrogen gas is adsorbed on the metal oxide, leading to a delay in response. Furthermore, because gas detection is carried out at relatively high temperatures, in elements containing a catalyst, such as Pd, in the metal oxide, the catalyst quickly deteriorates, thereby losing its catalytic activity.

Thus it has been desired to develop a hydrogen gas detecting element which can be used at lower temperatures and which has a shorts response time as well as high selectivity for hydrogen.

A carbon monoxide gas detecting apparatus to selectively detect carbon monoxide gas in exhaust gas from various apparatuses and a method of producing the apparatus are known as described in, for example, JP-A-59 119249. In accordance with this method, a mixture of stannic oxide and 2 to 10 mol% based on said stannic oxide of an aqueous platinum-containing chloroplatinic acid solution is freeze dried, antimony oxychloride is added to the dried mixture in an amount of 2 to 8 mol% based on the stannic oxide, the resulting mixture is added to an organic solvent to form a paste, and the paste is coated on an insulator equipped with an electrode and sintered in air to thereby produce a carbon monoxide gas detecting apparatus which can detect carbon monoxide at room temperature without heating the element.

Summary of the Invention

It has been found that if the Pt of the above carbon monoxide gas detecting element is replaced with Pd, or Pd is added to Pt and treated in a silane gas atmosphere having a low silane content, an element can be obtained which can selectively detect hydrogen at temperatures of the order of $300 ^\circ$ C and at high S/N ratio.

Accordingly, the present invention relates to a hydrogen gas detecting element which is produced by treating an element body comprising $SnO_2$ and Pd and Sb dispersed on said $SnO_2$, Pd/Sn being 0.1 to 8 mol% and Sb/Sn being 0 to 8 mol%, in a silane gas atmosphere having a silane content of 500 to 5,000 ppm, thereby dispersing $SiO_2$ on the element body.

Furthermore the present invention relates to a hydrogen gas detecting element which is produced by treating an element body comprising $SnO_2$, and Pd and Pt, or Pd, Pt and Sb dispersed on said $SnO_2$, Pd/Sn being 0.1 to 8 mol%, Pt/Sn being 0.5 to 8 mol% and Sb/Sn being 0 to 8 mol%, in a silane gas atmosphere having a silane content of 500 to 5,000 ppm, thereby dispersing a Si oxide on the element.

The present invention relates to a method of producing a hydrogen gas detecting element which comprises adding $SnO_2$ to a $PdCl_2$ solution prepared by adding $PdCl_2$, for example, to a 0.2% aqueous hydrochloric acid solution, in such an amount that the Pd/Sn ratio is 0.1 to 8 mol%, thoroughly dispersing $SnO_2$ in the above-obtained solution preferably with ultrasonic waves, quickly freeze drying the solution at a temperature of not higher than $-40 ^\circ$ C by the use of a vacuum freeze drier to obtain a dried product, adding SbOCl to the dried product in such an amount that the Sb/Sn molar ratio is 0 to 8 mol%, thoroughly mixing the resulting mixture in, for example, a mortar for about 30 minutes, for example, then adding the resulting mixture to an organic solvent, such as isopropyl alcohol to form a paste, coating and drying the paste on an alumina porcelain tube equipped with electrodes, sintering an element body having a $SnO_2$ and $PdCl_2$, or $SnO_2$, $PdCl_2$ and SbOCl-containing layer on the surface thereof in an air atmosphere or an antimony oxide gas atmosphere, providing the sintered element body with a heater, heating the sintered element body to 300 C° ± 50°C with the heater to age it for a predetermined time, and treating the element body by heating it to 300 to 350°C in a silane gas atmosphere having a silane content of 500 to 5,000 ppm to thereby disperse $SiO_2$ on the element body.

Furthermore the present invention relates to a method of producing a hydrogen gas detecting element which comprises preparing an aqueous solution of palladium chloride ($PdCl_2$), preparing an aqueous solution of chloroplatinic acid ($H_2PtCl_6$),adding the aqueous palladium chloride solution and the aqueous chloroplatinic acid solution in any desired order to stannic oxide in such amounts that the Pd/Sn ratio is 0.1 to 8 mol% and the Pt/Sn ratio is 0.5 to 8 mol%, thoroughly dispersing $SnO_2$ in the obtained mixture preferably with ultrasonic waves, drying the mixture to obtain a dried product, adding SbOCl to the dried product in such an amount that the Sb/Sn ratio is 0 to 8 mol% and sufficiently mixing them in, for example, a mortar for, for example, about 30 minutues, adding the mixture to an organic solvent, such as isopropyl alcohol to form a paste, coating and drying, the paste on an alumina porcelain tube equipped with electrodes, sintering an element body having a $SnO_2$, $PdCl_2$ and $H_2PtCl_6$, or $SnO_2$, $PdCl_2$, $H_2PtCl_6$ and SbOCl-containing layer on the surface thereof in an air atmosphere or an antimony oxide gas atmosphere, providing the sintered element body with a heater, heating the sintered element body to 300°C ± 50°C with the heater to age it in air for a predetermined time, and treating the element body by heating it at 300 to 350°C in a silane gas atmosphere having a silane content of 500 to 5,000 ppm to thereby disperse $SiO_2$ on the element body.

Brief Description of Drawings

Fig. 1 and 2 show the response characteristics of the hydrogen gas detecting element of Examples 10 and 38 of the present invention;

Fig. 3 and 4 are models showing the dispersion state of Si; and

Fig. 5 shows a relation between the amount of dichlorosilane at the time of silane gas treatment and the SN ratio.

Detailed Description of Preferred Embodiment

As an acid to be used in the preparation of the $PdCl_2$ solution, hydrochloric acid is preferred in that it has the same anion as $PdCl_2$. The concentration of the acid need only be sufficient to permit dissolution of $PdCl_2$. If the Pd/Sn ratio is less than 0.1 or more than 8 mol%, a hydrogen detecting ability undesirably drops. More preferably Pt is added in such an amount that the Pt/Sn ratio is within the range of 0.1 to 8 mol%. If $SnO_2$ is added to the $PdCl_2$ solution, preferably the $PdCl_2$ and $H_2PtCl_6$ solution as prepared above and well dispersed therein by the use of e.g., ultrasonic waves and, thereafter, the mixture is quickly freeze dried at a temperature of not higher than -40°C by the use of a vacuum freeze drier, the yield of the product is increased. The element body can be produced without application of the vacuum freeze drying, but in this case, however, the yield of the product is decreased. As organic solvents to form a paste of a mixture of the dried product and antimony oxychloride, as well as isopropyl alcohol, organic solvents such as a solvent of 25 wt% of $\beta$-terpineol, 72 wt% of butylcarbitol acatate and 3 wt% of ethyl cellulose can be used. As a base on which the paste is to be coated, as well as a porcelain tube, a tubular or board insulator capable of withstanding sintering can be used. The paste may be allowed to dry for about several minutes, or may be dried by the use of e.g., a thermostat.

The antimony oxide gas atmosphere is prepared by burning 0.5 to 7.5 mg ($2 \times 10^{-9}$ to $3 \times 10^{-8}$ mol/cm³ as $Sb_2O_3$) of SbOCl or $Sb_2O_3$ at 600 to 850°C for about 5 to 30 minutes. If the amount of the SbOCl used is in excess of 7.5mg, Sb covers Pd and Pt, thereby decreasing the activity of Pd and Pt. The element body is sintered in an air atmosphere or an antimony oxide atmosphere at a temperature of 600 to 850°C for 5 to 60 minutes. If the sintering is carried out at temperatures of less than 600°C for less than 5 minutes, antimony cannot be doped satisfactorily. At temperatures higher than 850°C, the activities of Pd and Pt drop and thus hydrogen cannot be detected selectively.

Aging of the element body is carried out by heating the element body to 300 ± 50°C by the use of a heater provided to the element body and heating it in an air atmosphere for not less than 12 hours. This aging stabilizes a semiconductor layer. The treatment of the element body in a silane gas-containing air atmosphere is carried out by heating the element at 300 to 350°C for 5 to 45 minutes in a dichlorosilane ($SiH_2Cl_2$) gas-containing air atmosphere or monosilane ($SiH_4$) gas-containing air atmosphere having a concentration of 500 to 5,000 ppm by the use of a heater. If the silane gas concentration and the treating time are not within the above specified ranges, the hydrogen detecting sensitivity drops. The element body is further heated at 300 ± 50°C in air for not less than 12 hours to produce a final hydrogen gas detecting element. By repeating the treatment in a silane gas-containing air atmosphere and the subsequent heat treatment in air in the same order, the selectivity to hydrogen gas can be increased even more.

In the present invention, Si is dispersed on the surface of the element body upon treatment of the element body in the presence of a small amount of silane gas. As apparent from the examples as described hereinafter, the operation temperature is on the level of 300°C and the response speed is not more than 50 seconds.

The present invention is described in greater detail with reference to the following examples.

## EXAMPLES 1 TO 9

SnO_2 was well dispersed with ultrasonic waves in a $2 \times 10^{-2}$ mol/l conc. solution of PdCl_2 in 0.2 % conc. hydrochloric acid in the amount shown in Table 1 so that the Pd/Sn molar ratio was as specified in the table. This aqueous dispersion was placed in a vacuum freeze drier and quickly freeze dried at -40°C. Then SbOCl was added to the dried product in such an amount that the Sb/Sn ratio was 1.0 mol% and mixed for about 30 minutes in a mortar. To the resulting mixture was added isopropyl alcohol to form a paste. This paste was coated on an alumina porcelain tube equipped with an electrode and was allowed to dry. Then the element body was sintered at 700°C for 15 minutes. This sintering was conducted in an Sb oxide gas atmosphere which had been prepared by sintering 2.4 mg of SbOCl in a quartz tube. A heater was provided to the sintered element body. Then the element body was heated to 300°C by passing electricity through the heater and aged in air for 12 hours to stabilize a semiconductor layer. The element body was then heated to 325°C and treated only once for 10 minutes in a 500 ppm dichlorosilane-containing air atmosphere. The thus obtained hydrogen detecting element had an electric resistance (kΩ) and SN ratio (resistance value of the element in pure air/resistance value of the element in feed gas) measured at a temperature of 325°C ± 10°C in a feed gas concentration of 100 ppm (ordinary temperature) as shown in Table 1. In Table 1, Example 1 indicates a comparative example in which an element body was not subjected to silane treatment, and Examples 2 and 3 are comparative examples in which elements have a Pd/Sn molar ratio not falling within the range specified in the present invention. In Examples 3 to 9, the elements exhibit sensitivity to isopropyl alcohol (i-PA) but have high hydrogen selectivity.

In each example of Table 1, either 4 or 8 elements having the same composition were prepared and the average value of 4 or 8 elements is shown. This is the same in Tables 2 to 4 as described hereinafter.

Table 1

| Ex No. | Pd/Sn (mol %) | Sb/Sn (mol %) | Atmosphere for sintering | SiH₂Cl₂ (ppm-min) | Air | H₂ | CH₄ | C₂H₄ | C₂H₆ | iC₄H₁₀ | CO | NH₃ | iPA |
|---|---|---|---|---|---|---|---|---|---|---|---|---|---|
| 1 | 1.0 | 1.0 | 2.4mg SbOCl | | 17 | 4.8 | | 3.4 | 8.9 | 5.9 | 7.8 | 6.6 | 2.9 |
|   |     |     |             |        | *  | 3.54 |     | 5.00 | 1.91 | 2.88 | 2.18 | 2.56 | 5.86 |
| 2 | 0.025 | 0 | 2.4mg SbOCl | 500—10 | 1.61 | 0.577 | 1.58 | 1.45 | 1.56 | 1.59 | 1.38 | 1.36 | 0.62 |
|   |       |   |             |        | *    | 2.79  | 1.02 | 1.11 | 1.03 | 1.01 | 1.17 | 1.18 | 2.61 |
| 3 | 0.05 | 1.0 | 2.4mg SbOCl | 500—10 | 0.647 | 0.250 | 0.628 | 0.599 | 0.599 | 0.634 | 0.553 | 0.549 | 0.324 |
|   |      |     |             |        | *     | 2.59  | 1.03  | 1.08  | 1.08  | 1.02  | 1.17  | 1.18  | 2.00 |
| 4 | 0.1 | 1.0 | 2.4mg SbOCl | 500—10 | 1.08 | 0.343 | 1.07 | 0.893 | 1.06 | 1.08 | 0.844 | | 0.593 |
|   |     |     |             |        | *    | 3.15  | 1.01 | 1.21  | 1.02 | 1.00 | 1.28  | | 1.82 |
| 5 | 0.4 | 1.0 | 2.4mg SbOCl | 500—10 | 3.22 | 0.591 | 3.19 | 1.46 | 3.13 | 3.19 | 1.75 | | 1.14 |
|   |     |     |             |        | *    | 5.45  | 1.01 | 2.21 | 1.03 | 1.01 | 1.84 | | 2.82 |
| 6 | 1.0 | 1.0 | 2.4mg SbOCl | 500—10 | 7.13 | 1.25 | 7.13 | 2.04 | 6.25 | 5.84 | 3.21 | 5.09 | 1.76 |
|   |     |     |             |        | *    | 5.71 | 1.00 | 3.50 | 1.14 | 1.22 | 2.22 | 1.40 | 4.04 |
| 7 | 2.0 | 1.0 | 2.4mg SbOCl | 500—10 | 30.1 | 5.68 | 30.1 | 12.3 | 27.6 | 26.9 | 14.5 | 21.9 | 6.72 |
|   |     |     |             |        | *    | 5.30 | 1.00 | 2.44 | 1.09 | 1.12 | 2.08 | 1.37 | 4.48 |
| 8 | 4.0 | 1.0 | 2.4mg SbOCl | 500—10 | 7.5 | 1.15 | 7.35 | 1.95 | 6.52 | 6.10 | 3.32 | 4.60 | 1.49 |
|   |     |     |             |        | *   | 6.50 | 1.02 | 3.85 | 1.15 | 1.23 | 2.26 | 1.63 | 5.04 |
| 9 | 6.0 | 1.0 | 2.4mg SbOCl | 500—10 | 16.9 | 2.81 | 16.7 | 6.01 | 15.80 | 15.23 | 8.24 | 11.1 | 4.31 |
|   |     |     |             |        | *    | 6.02 | 1.01 | 2.81 | 1.07  | 1.11  | 2.05 | 1.52 | 3.92 |

Resistance kΩ (SN ratio) Upper/Lower

Feed gas conc. 100ppm (ordinary temp)
Temp of elements while detecting 325±10°C

## EXAMPLES 10 to 20

Elements of Examples 10 to 20 were produced in the same manner as in Examples 2 to 9 except that the Pd/Sn molar ratio and Sb/Sn molar ratio shown in Table 2 were used and the amount of dichlorosilane used was 1,000 ppm. The resistance value (kΩ) and the SN ratio of each element to feed gases as determined in a feed gas-containing air atmosphere having a feed gas concentration of 100 ppm are shown in Table 2. The temperature of the element at measurement was 325°C ± 10°C. In Examples 10 to 13, the Sb/Sn ratio was maintained at 0.5 mol% and the Pd/Sn molar ratio alone was changed. The SN ratio of hydrogen gas was not less than 2 times that of the other gas. This indicates that within this range, the Pd/Sn molar ratio does not exert any influence. In Examples 14 to 17, the Sb/Sn ratio was maintained at 2.0 mol%, but the Pd/Sn ratio was changed from 0.5 to 8 mol%. The SN ratio of hydrogen gas was not less than 4 times that of other gas, and the hydrogen selectivity was increased more than in Examples 10 to 13. Example 20 indicates a case in which the amounts of Pd and Sb were set at the upper limits. The SN ratio of hydrogen gas was 3.7 times that of other gas, but the hydrogen selectivity was decreased as compared with Examples 14 to 17. In Examples 18 and 19, the Pd/Sn ratio was maintained at 4.0 mol% and the Sb/Sn molar ratio was changed and further the treating time in dichlorosilane was changed to 20 minutes. In these examples, the SN ratio of hydrogen gas was not less than 2.5 times that of other gas.

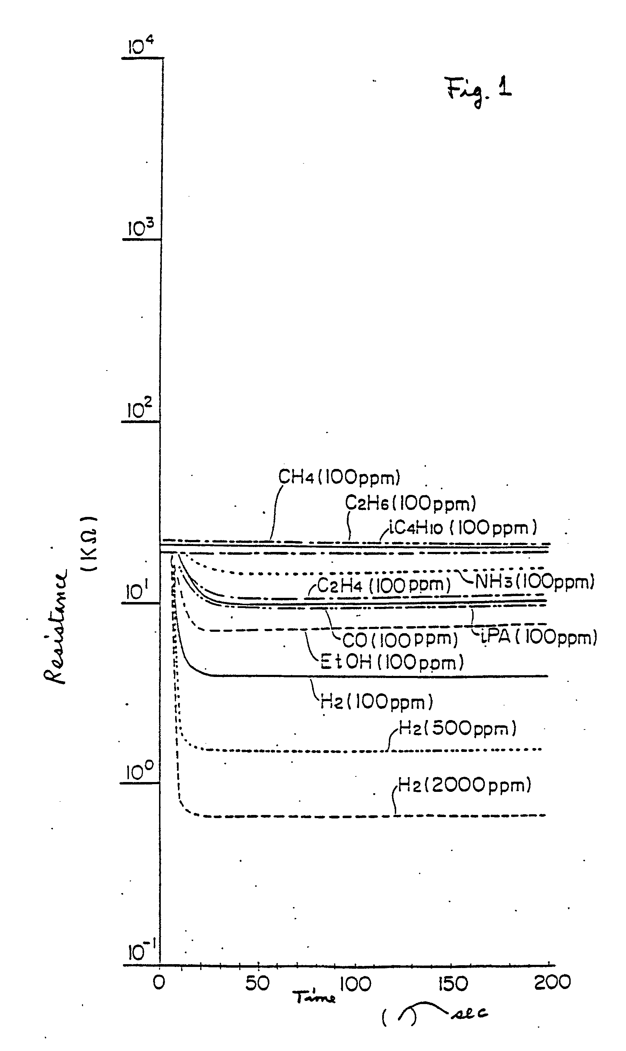

The response characteristics of one of the elements of Example 10 to each gas sample are shown in Fig. 1. It can be seen from Fig. 1 that the response time to hydrogen gas is short. This is beleived due to the quick adsorption of hydrogen gas because $SiO_2$ is dispersed on the element surface.

Table 2

| Ex No. | Pd/Sn (mol %) | Sb/Sn (mol %) | Atmosphere for sintering | $SiH_2Cl_2$ (ppm-min) | Air | $H_2$ | $CH_4$ | $C_2H_4$ | $C_2H_6$ | $iC_4H_{10}$ | $CO$ | $NH_3$ | iPA | EtOH |
|---|---|---|---|---|---|---|---|---|---|---|---|---|---|---|
| 1 | 1.0 | 1.0 | 2.4mg SbOCl | | 17 / * | 4.8 / 3.54 | | 3.4 / 5.00 | 8.9 / 1.91 | 5.9 / 2.88 | 7.8 / 2.18 | 6.6 / 2.56 | 2.9 / 5.86 | |
| 10 | 1.0 | 0.5 | 2.5mg SbOCl | 1000-10 | 23 / * | 4.5 / 5.11 | 23 / 1.00 | 17 / 1.35 | 22 / 1.04 | 21 / 1.09 | 11 / 2.09 | | 14 / 1.64 | 9.8 / 2.35 |
| 11 | 2.0 | 0.5 | 2.5mg SbOCl | 1000-10 | 100 / * | 9.2 / 10.9 | | | | | | | 70 / 1.43 | |
| 12 | 4.0 | 0.5 | 2.5mg SbOCl | 1000-10 | 110 / * | 12 / 9.17 | | | | | | | 96 / 1.15 | |
| 13 | 6.0 | 0.5 | 2.5mg SbOCl | 1000-10 | 36 / * | 4.2 / 8.57 | | | | | | | 29 / 1.24 | |
| 14 | 0.5 | 2.0 | 2.5mg SbOCl | 1000-10 | 3.5 / * | 0.51 / 6.86 | | 3.0 / 1.17 | | 3.2 / 1.09 | 2.4 / 1.46 | 2.6 / 1.35 | 3.0 / 1.17 | 2.8 / 1.25 |
| 15 | 1.0 | 2.0 | 2.5mg SbOCl | 1000-10 | 35 / * | 4.2 / 3.33 | | 30 / 1.17 | | 31 / 1.13 | 26 / 1.35 | 28 / 1.25 | 28 / 1.25 | 21 / 1.57 |
| 16 | 2.0 | 2.0 | 2.5mg SbOCl | 1000-10 | 10 / * | 1.3 / 7.69 | | 9 / 1.11 | | 9 / 1.11 | 8 / 1.25 | 8 / 1.25 | 9 / 1.11 | |
| 17 | 8.0 | 2.0 | 2.5mg SbOCl | 1000-10 | 3.3 / * | 0.55 / 6.00 | | 2.7 / 1.22 | | 2.9 / 1.14 | 2.3 / 1.43 | 2.5 / 1.32 | 2.6 / 1.27 | |
| 18 | 4.0 | 2.0 | 2.5mg SbOCl | 1000-20 | 33 / * | 4.0 / 8.25 | 30 / 1.10 | 30 / 1.10 | 30 / 1.10 | 30 / 1.10 | 23 / 1.43 | 11 / 3.3 | | |
| 19 | 4.0 | 4.0 | 2.5mg SbOCl | 1000-20 | 27 / * | 3.6 / 7.50 | 27 / 1.00 | 22 / 1.22 | 25 / 1.03 | 25 / 4.08 | 21 / 1.29 | 15 / 1.80 | 23 / 1.17 | 14 / 1.93 |
| 20 | 8.0 | 8.0 | 2.5mg SbOCl | 1000-10 | 14.8 / * | 2.7 / 5.56 | | 12.1 / 1.22 | 14.8 / 1.00 | 14.6 / 1.01 | 12.4 / 1.19 | 10.2 / 1.45 | 9.9 / 1.49 | |

Upper resistance kΩ
Lower (SN ratio)

Feed gas conc. (ordinary temp)
Temp of elements while detecting 325±10°C

## EXAMPLES 21 TO 28

Examples 21 to 28 were conducted in the same manner as in the preceding examples except that the treatment using dichlorosilane was conducted for 10 minutes in a 500 ppm dichlorosilane-containing air atmosphere and then for 10 minutes in a 1,000 ppm dichlorosilane-containing air atmosphere. In the table, Examples 1, 21 and 22 are comparative examples. By comparison of the results of Example 6 and those of Example 25, it can be seen that the SN ratio was increased by conducting the dichlorosilane treatment twice. Also, by comparision of the results of Example 1 with those of Example 25, it can be seen that the sensitivity to gases except for hydrogen gas is almost lost. Also by comparing the results of Table 2 with those of Table 3, it can be seen that if the treatment in a silane gas atmosphere is carried out twice for a short time in place of conducting the treatment once for a long time, the hydrogen gas selectivity is increased even more.

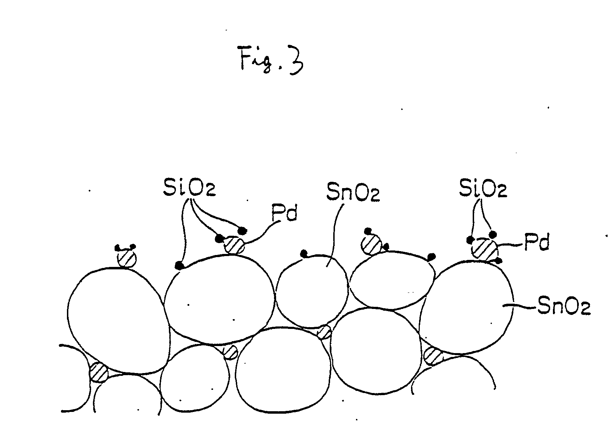

In the element of Sample 25, the amount of Si dispersed was calculated and assumed to be about 0.5 wt%. The Pd and Si dispersion state on the surface of one element of Example 25 was measured by an electron probe microanalyzer (3000 x). This analysis confirms that Pd is dispersed on $SnO_2$ and Si is dispersed in a dispersion state almost corresponding to the dispersion state of Pd. Accordingly it is felt that the major portion of Si is dispersed on the Pd surface as shown in the model of Fig. 3. As a result, the oxidation catalytic ability of Pd towards gases other than hydrogen gas is lost and almost no adsorption of other gases other than hydrogen gas occurs. On the other hand, hydrogen gas is rapidly adsorbed on the element surface without being disturbed. As a result, as shown by the characteristics of one element of Example 10 in Fig. 1, the response to hydrogen is satisfactory.

Table 3

Upper   resistance k Ω
Lower   (SN ratio)

| Ex No. | Pd/Sn (mol %) | Sb/Sn (mol %) | Atmosphere for sintering | SiH₂Cl₂ (ppm-min) | Air | H₂ | CH₄ | C₂H₄ | C₂H₆ | iC₄H₁₀ | CO | NH₃ | iPA |
|---|---|---|---|---|---|---|---|---|---|---|---|---|---|
| 1 | 1.0 | 1.0 | 2.4 mg SbOCl |  | 17 | 4.8 |  | 3.4 | 8.9 | 5.9 | 7.8 | 6.6 | 2.9 |
|  |  |  |  |  | * | 3.54 |  | 5.00 | 1.91 | 2.88 | 2.18 | 2.56 | 5.86 |
| 21 | 0.025 | 1.0 | 2.4 mg SbOCl | 500-10 | 3.0 | 0.74 |  | 3.0 | 3.0 | 3.0 | 3.0 | 3.0 | 2.2 |
|  |  |  |  | 1000-10 | * | 6.38 |  | 1.00 | 1.00 | 1.00 | 1.00 | 1.00 | 1.36 |
| 22 | 0.05 | 1.0 | 2.4 mg SbOCl | 500-10 | 1.0 | 0.32 |  | 1.0 |  | 1.0 | 1.0 | 1.0 | 1.0 |
|  |  |  |  | 1000-10 | * | 3.12 |  | 1.00 |  | 1.00 | 1.00 | 1.00 | 1.00 |
| 23 | 0.1 | 1.0 | 2.4 mg SbOCl | 500-10 | 1.2 | 0.40 |  | 1.1 |  | 1.1 | 1.0 | 1.0 | 0.9 |
|  |  |  |  | 1000-10 | * | 3.00 |  | 1.09 |  | 1.09 | 1.20 | 1.20 | 1.33 |
| 24 | 0.4 | 1.0 | 2.4 mg SbOCl | 500-10 | 4.0 | 0.45 |  | 3.7 |  | 3.7 | 2.4 | 3.0 | 3.0 |
|  |  |  |  | 1000-10 | * | 8.89 |  | 1.08 |  | 1.08 | 1.67 | 1.33 | 1.33 |
| 25 | 1.0 | 1.0 | 2.4 mg SbOCl | 500-10 | 11.0 | 1.2 |  | 11.0 |  | 11.0 | 7.5 | 9.5 | 9.5 |
|  |  |  |  | 1000-10 | * | 9.17 |  | 1.00 |  | 1.00 | 1.47 | 1.16 | 1.16 |
| 26 | 2.0 | 1.0 | 2.4 mg SbOCl | 500-10 | 37.0 | 2.9 |  | 20.0 |  | 37 | 22 | 20 | 20 |
|  |  |  |  | 1000-10 | * | 12.76 |  | 1.85 |  | 1.00 | 1.68 | 1.85 | 1.85 |
| 27 | 4.0 | 1.0 | 2.4 mg SbOCl | 500-10 | 13.0 | 1.4 |  | 12 |  | 13 | 7.7 | 9.2 | 12 |
|  |  |  |  | 1000-10 | * | 9.28 |  | 1.08 |  | 1.00 | 1.69 | 1.41 | 1.08 |
| 28 | 6.0 | 1.0 | 2.4 mg SbOCl | 500-10 | 40.0 | 4.2 |  | 33 |  | 40 | 25 | 29 | 39 |
|  |  |  |  | 1000-10 |  | 9.52 |  | 1.21 |  | 1.00 | 1.60 | 1.38 | 1.02 |

Feed gas conc.  100ppm (ordinary temp)
Temp of elements while detecting  325±10°C

EXAMPLES 29 TO 32

Examples 29 to 32 are examples in which SbOCl was not mixed and the element body was sintered in an air atmosphere. This operation was conducted in the same manner as in Example 2 to 9. By comparison of Example 30 with Examples 11 and 16, Example 31 with Examples 12 and 19, and Example 32 with Example 13 in connection with the SN value of $H_2$ as shown in Table 4, it can be seen that almost equal hydrogen selectivity can be obtained.

Table 4

Upper resistance kΩ
Lower (SN ratio)

| Ex No. | Pd/Sn (mol %) | Sb/Sn (mol %) | Atmosphere for sintering | $SiH_2Cl_2$ (ppm-min) | Air | $H_2$ | $CH_4$ | $C_2H_4$ | $C_2H_6$ | $iC_4H_{10}$ | CO | $NH_3$ | iPA |
|---|---|---|---|---|---|---|---|---|---|---|---|---|---|
| 1 | 1.0 | 1.0 | 2.4 mg SbOCl | | 17 / * | 4.8 / 3.54 | | 3.4 / 5.00 | 8.9 / 1.91 | 5.9 / 2.88 | 7.8 / 2.18 | 6.6 / 2.56 | 2.9 / 5.86 |
| 29 | 0.4 | 0 | Air | 1000—10 | 130 / * | 15 / 8.67 | | 98 / 1.33 | 130 / 1.00 | 130 / 1.00 | 70 / 1.86 | 100 / 1.30 | 88 / 1.48 |
| 30 | 2.0 | 0 | Air | 1000—10 | 1600 / * | 190 / 8.42 | | 1100 / 1.45 | 1600 / 1.00 | 1600 / 1.00 | 850 / 1.88 | 1000 / 1.60 | 1000 / 1.60 |
| 31 | 4.0 | 0 | Air | 1000—10 | 250 / * | 28 / 8.93 | | 90 / 2.78 | 230 / 1.09 | 230 / 1.09 | 125 / 2.00 | 130 / 1.92 | 55 / 4.54 |
| 32 | 6.0 | 0 | Air | 1000—10 | 290 / * | 33 / 8.79 | | 130 / 2.23 | 280 / 1.03 | 280 / 1.03 | 150 / 1.93 | 150 / 1.93 | 100 / 2.90 |

Feed gas conc. 100ppm (ordinary temp)
Temp of elements while detecting 325±10°C

10

EXAMPLES 33 TO 45

A 2x10$^{-2}$ mol/l conc. solution of PdCl$_2$ in 0.2% hydrochloric acid and a 5x10$^{-2}$ mol/l conc. solution of H$_2$PtCl$_6$ in pure water in the amounts shown in Table 5 were succesivly added to SnO$_2$ and SnO$_2$ in this mixture was well dispersed with ultrasonic waves. This dispersion was quickly freeze dried at -40°C, and placed in a vacuum freeze drier and dried. SbOCl was added to the above dried product in such an amount that the Sb/Sn molar ratio shown was obtained and mixed in a mortar for about 30 minutes. To this mixture was added isopropyl alcohol to form a paste. This paste was coated on an alumina porcelain tube equipped with electrodes and then was allowed to dry. This element body was sintered for 15 minutes at 700°C in a quartz tube in an antimony oxide gas atmosphere which had been prepared by burning 2.5 mg of SbOCl. The sintered element body was provided with a heater. Then the element body was heated to 300°C by passing electricity through the heater and then aged in air for 12 hours. In the examples other than Example 33 (comparative example), the element body was treated once for 10 minutes in a 1,000 ppm dichlorosilane-containing air atmosphere and then aged in air for 12 hours while being heating at 300°C. The hydrogen gas detecting element thus obtained was measured for electric resistance (kΩ) and SN ratio (resistance value of the element in pure air/resistance value of the element in feed gas) measured at a temperature of 325°C ± 10°C in air containing 100 ppm of each feed gas (at ordinary temperature), and also the response (seconds). The results are shown in Table 5. In Table 5, Example 33 indicates a comparative example in which an element was not subjected to silane treatment, and Examples 34 to 45 are examples of the present invention. In Examples 34 to 45, the response time was within 30 seconds. In Example 45, the amounts of Pd, Pt and Sb added were the upper limits. The figures shown in Table 5 indicate an average value of 8 samples produced in each sample.

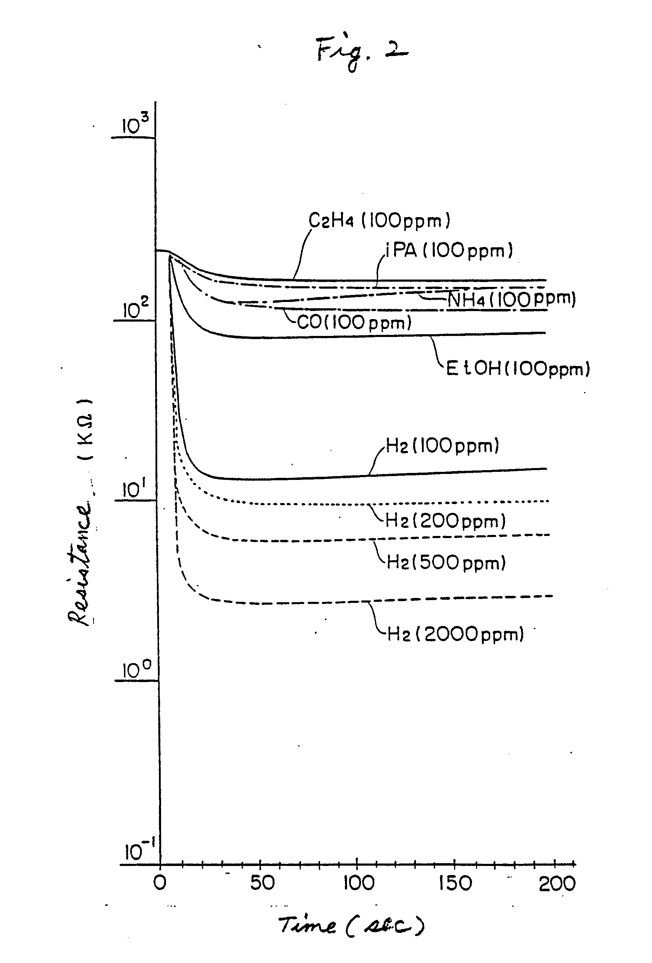

The response characteristics to each sample gas of one of the elements produced in Example 38 are shown in Fig. 2.

Table 5

Upper   resistance kΩ
Middle  SN ratio
Lower   response time (sec)

| Ex No. | Pd/Sn (mol %) | Pt/Sn (mol %) | Sb/Sn (mol %) | Atmosphere for sintering | $SiH_2Cl_2$ (ppm-min) | Air | $H_2$ | $CH_4$ | $C_2H_4$ | $C_2H_6$ | $iC_4H_{10}$ | CO | $NH_3$ | EtOH | iPA |
|---|---|---|---|---|---|---|---|---|---|---|---|---|---|---|---|
| 33 | 1 | 0.5 | 0.5 | SbOCl 2.5mg | | 462 | 92.8 | 350 | 93.9 | 193 | 116 | 254 | 350 | 66.1 | 71.6 |
| | | | | | | * | 4.98 | 1.32 | 4.92 | 2.40 | 4.00 | 1.82 | 1.32 | 6.99 | 6.45 |
| | | | | | | * | 50.2 | 119 | 20.3 | 60.7 | 28.6 | 172 | 27.6 | 21.2 | 41.0 |
| 34 | 0.1 | 1 | 1 | SbOCl 2.5mg | 1000-10 | 93.6 | 20.5 | | 41.2 | 85.1 | 71.5 | 60.0 | 34.8 | | 15.6 |
| | | | | | | * | 4.57 | | 2.27 | 1.10 | 1.31 | 1.56 | 2.69 | | 5.99 |
| | | | | | | * | 17 | | 18 | | 134 | 24 | 23 | | 17 |
| 35 | 1 | 0.5 | 0.5 | SbOCl 2.5mg | 1000-10 | 91.0 | 8.85 | | 36.8 | | 82.7 | 31.8 | 51.4 | 15.7 | 23.5 |
| | | | | | | * | 10.2 | | 2.47 | | 1.10 | 2.86 | 1.77 | 5.81 | 3.88 |
| | | | | | | * | 19.0 | | 41.4 | | 66.6 | 41.5 | 27.5 | -20.2 | 29.5 |
| 36 | 1 | 0.5 | 1 | SbOCl 2.5mg | 1000-10 | 54.1 | 6.54 | | 40.4 | | | | | 21.6 | |
| | | | | | | * | 8.28 | | 1.34 | | | | | 2.51 | |
| | | | | | | * | 19.2 | | 40.8 | | | | | 20.3 | |
| 37 | 1 | 1 | 0.5 | SbOCl 2.5mg | 1000-10 | 95.0 | 10.6 | | 29.7 | | 70.4 | 36.7 | 59.4 | 16.7 | 20.1 |
| | | | | | | * | 8.87 | | 3.20 | | 1.35 | 2.59 | 1.60 | 5.68 | 4.72 |
| | | | | | | * | 19.3 | | 42.9 | | 120 | 54.3 | 32.8 | 24.1 | 30.3 |
| 38 | 1 | 2 | 0.5 | SbOCl 2.5mg | 1000-10 | 167 | 15.6 | | 63.0 | | 137 | 69.0 | 97.7 | 23.6 | 37.4 |
| | | | | | | * | 10.72 | | 2.65 | | 1.22 | 2.42 | 1.71 | 7.08 | 4.47 |
| | | | | | | * | 18.5 | | 42.1 | | 102 | 56.7 | 24.4 | 34.1 | 65.0 |
| 39 | 2 | 4 | 0.5 | SbOCl 2.5mg | 1000-10 | 28.4 | 3.59 | 28.4 | 22.4 | 28.4 | 28.4 | 20.1 | 21.8 | 12.9 | 22.9 |
| | | | | | | * | 7.91 | 1.00 | 1.27 | 1.00 | 1.00 | 1.41 | 1.30 | 2.21 | 1.24 |
| | | | | | | * | 29.8 | | 120 | | | 297 | 62.6 | 69.9 | 66.8 |
| 40 | 4 | 4 | 0.5 | SbOCl 2.5mg | 1000-10 | 20.6 | 3.03 | 20.3 | 11.4 | 19.6 | 19.3 | 13.6 | 15.9 | 7.03 | 12.3 |
| | | | | | | * | 6.84 | 1.01 | 1.89 | 1.05 | 1.07 | 1.51 | 1.29 | 2.93 | 1.68 |
| | | | | | | * | 19.7 | | 103 | | | 224 | 88.4 | 55.4 | 107 |

EXAMPLE 46

The operation was conducted in the same manner as in Examples 34 to 45 except that the SiH$_2$Cl$_2$ treatment was conducted at 1,000 ppm for 10 minutes at the first stage and at 1,000 ppm for 10 minutes at

Table 5 (continued)

Upper   resistance kΩ
Middle  SN ratio
Lower   response time (sec)

| Ex No. | Pd/Sn (mol %) | Pt/Sn (mol %) | Sb/Sn (mol %) | Atmosphere for sintering | SiH$_2$Cl$_2$ (ppm-min) | Air | H$_2$ | CH$_4$ | C$_2$H$_4$ | C$_2$H$_6$ | iC$_4$H$_{10}$ | CO | NH$_3$ | EtOH | iPA |
|---|---|---|---|---|---|---|---|---|---|---|---|---|---|---|---|
| 41 | 4 | 4 | 4 | SbOCl 2.5mg | 1000−10 | 31.3 | 5.10 | | 27.9 | 31.0 | 31.0 | 27.5 | 31.3 | 26.3 | 27.9 |
| | | | | | | * | 6.1 | | 1.12 | 1.01 | 1.01 | 1.14 | 1.00 | 1.19 | 1.12 |
| | | | | | | * | 42 | | 61 | | | 102 | | 65 | 116 |
| 42 | 4 | 4 | 8 | SbOCl 2.5mg | 1000−10 | 15.6 | 2.80 | | 15.4 | 15.6 | 15.6 | 15.4 | 15.6 | 13.1 | 15.1 |
| | | | | | | * | 5.58 | | 1.01 | 1.00 | 1.00 | 1.01 | 1.00 | 1.19 | 1.03 |
| | | | | | | * | 74 | | | | | | | 32 | 0 |
| 43 | 6 | 1 | 0.5 | SbOCl 2.5mg | 1000−10 | 15.5 | 1.14 | 15.5 | 11.3 | 15.5 | 15.5 | 10.5 | 11.2 | 4.39 | 11.3 |
| | | | | | | * | 12.7 | 1.00 | 1.97 | 1.00 | 1.00 | 1.48 | 1.38 | 3.53 | 1.37 |
| | | | | | | * | 96.6 | | 130 | | | 295 | 90.3 | 119 | 110 |
| 44 | 6 | 6 | 0.75 | SbOCl 2.5mg | 1000−10 | 53.4 | 5.03 | 51.3 | 25.9 | 49.4 | 47.7 | 37.9 | 32.2 | 17.5 | 32.6 |
| | | | | | | * | 10.1 | 1.04 | 2.06 | 1.08 | 1.12 | 1.41 | 1.66 | 3.06 | 1.64 |
| | | | | | | * | 13.2 | | 102 | | | 261 | 68.3 | 40.7 | 82.6 |
| 45 | 8 | 8 | 8 | SbOCl 2.5mg | 1000−10 | 8.85 | 1.58 | | 2.59 | 7.02 | 6.86 | 8.12 | 8.76 | | 1.98 |
| | | | | | | * | 5.95 | | 3.41 | 1.26 | 1.29 | 1.09 | 1.01 | | 4.46 |
| | | | | | | * | 55 | | 110 | 96 | 56 | | | | 95 |

Feed gas conc.  100ppm  (ordinary temp)
Temp of elements while detecting  325±10°C

13

the second stage. The characteristics of the element produced are shown in Table 6 in comparison with the characteristics of the elements of Examples 33 to 35. In Table 6, it is shown that the SN ratio to hydrogen gas of the element of Example 46 is greatly increased as compared with that of the element of Example 35 while on the other hand the SN ratio to other gases of the element of Example 46 is decreased as compared with that of the element of Example 35. Hence it is understood that a hydrogen gas selectivity was significantly improved when element body was treated by $SiH_2Cl_2$ two or more times. The temperature of the element at the time of measurement was $325°C$ $10°C$.

EXAMPLE 47

An element was produced in the same manner as in Examples 34 to 45 except that antimony oxychloride was not added and the treatment in an antimony oxychloride oxidizing atmosphere was not applied. The response time of the element of Example 47 was 37 seconds, which was somewhat longer than those of the elements of Examples 34 to 45. However the response of the element of Example 47 was much more rapid that the element in which only Pt was added, and further the element was good in hydrogen selectivity. Production conditions and characteristics of the elements of Examples 46 and 47 are shown in Table 6.

In one of the elements of Example 46, the amount of Si dispersed was calculated and assumed to be 0.5 wt%.

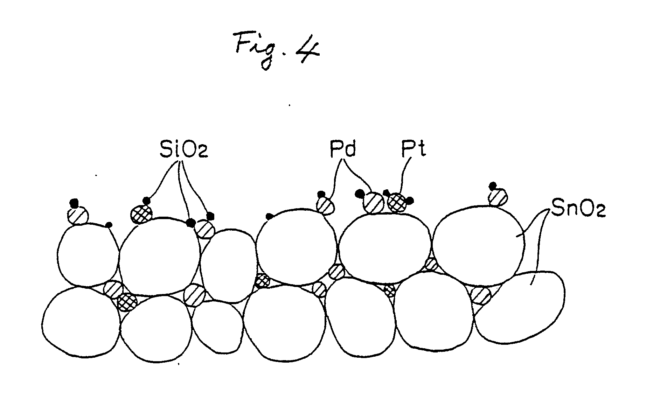

For one of the elements of Example 46, the dispersion state of Pt, Pd and Si on the element surface was measured with an electron probe microanalyzer (3000 x). It was confirmed that Pt and Pd were dispersed on $SnO_2$ (Pt was only slightly dispersed because the amount of Pt was as low as 0.5 mol% based on Sn), and Si was dispersed in a dispersion form almost corresponding to the dispersion state of Pd and Pt. It is therefore considered that the major portion of Si is dispersed on the surface of Pd and Pt as shown in the model of Fig. 4. As a result, it is considered that the transfer of electrons between gases other than hydrogen gas and the element surface becomes very difficult and thus the SN ratio drops, while on the other hand since the adsorption of hydrogen gas onto the element surface and the electron transfer are carried out smoothly and thus the SN ratio is increased and the response is rapid.

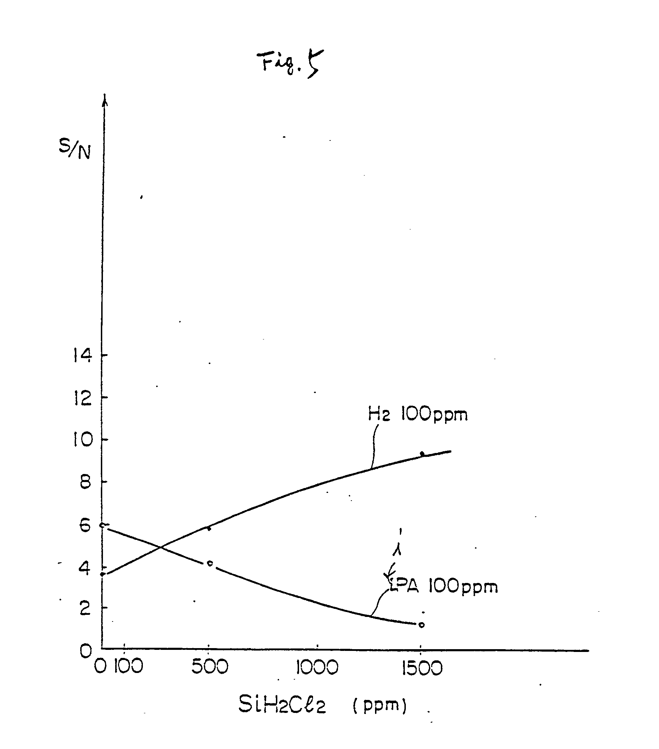

The relation between the amount (ppm) of $SiH_2Cl_2$ and the SN ratio when an element body is treated with silane gas, the element body being produced under the conditions that the Pd/Sn molar ratio was 1 and the Sb/Sn molar ratio was 1 is shown in Fig. 5.

| Ex No. | Pd/Sn (mol %) | Pt/Sn (mol %) | Sb/Sn (mol %) | Atmosphere for sintering | SiH2Cl2 (ppm-min) | Air | H2 | CH4 | C2H4 | C2H6 | iC4H10 | CO | NH3 | EtOH | iPA |
|---|---|---|---|---|---|---|---|---|---|---|---|---|---|---|---|
| 33 | 1 | 0.5 | 0.5 | SbOCl 2.5mg | none | 462 | 92.8 | 350 | 93.9 | 193 | 116 | 254 | 350 | 66.1 | 71.6 |
|  |  |  |  |  |  | * | 4.98 | 1.32 | 4.92 | 2.40 | 4.00 | 1.82 | 1.32 | 6.99 | 6.45 |
|  |  |  |  |  |  | * | 50.2 | 119 | 20.3 | 60.7 | 28.6 | 172 | 27.6 | 21.2 | 41.0 |
| 35 | 1 | 0.5 | 0.5 | SbOCl 2.5mg | 1000—10 | 91.0 | 8.85 |  | 36.8 |  | 82.7 | 31.8 | 51.4 | 15.7 | 23.5 |
|  |  |  |  |  |  | * | 10.2 |  | 2.47 |  | 1.10 | 2.86 | 1.77 | 5.81 | 3.88 |
|  |  |  |  |  |  | * | 19.0 |  | 41.4 |  | 66.6 | 41.5 | 27.5 | 20.2 | 29.5 |
| 46 | 1 | 0.5 | 0.5 | SbOCl 2.5mg | 1st 1000—10 / 2nd 1000—10 | 133 | 6.09 |  | 93.7 |  |  | 64.3 |  | 60.5 | 85.8 |
|  |  |  |  |  |  | * | 22.1 |  | 1.42 |  |  | 2.07 |  | 2.20 | 1.55 |
|  |  |  |  |  |  | * | 19.4 |  | 65.9 |  |  | 64.2 |  | 35.3 | 60.4 |
| 47 | 1 | 1 | 0 | Air | 1000—10 | 660 | 77.2 |  | 516 | 641 | 524 | 391 | 434 |  | 317 |
|  |  |  |  |  |  | * | 8.85 |  | 1.28 | 1.03 | 1.26 | 1.69 | 1.52 |  | 2.08 |
|  |  |  |  |  |  | * | 37 |  | 121 |  | 163 | 288 | 277 |  | 255 |

Upper / Middle / Lower — resistance kΩ / SN ratio / response (sec)

Feed gas conc. 100ppm (ordinary temp)
Temp of elements while detecting 325±10°C

## Claims

1. A hydrogen gas detecting element produced by the following steps of dispersing Si oxide on an element body by treating said element body in a silane gas atmosphere having a silane content of 500

15

to 5,000 ppm, said element body containing Pd or Pd and Pt, or Pd, Pt and Sb dispersed on $SnO_2$, Pd/Sn being 0.1 to 8 mol%, Pt/Sn being 0 to 8 mol% and Sb/Sn being 0 to 8 mol%.

2. The hydrogen gas detecting element as claimed in Claim 1, wherein said element body comprises Pd or Pd and Sb dispersed on $SnO_2$, Pd/Sn being 0.1 to 8 mol%, Sb/Sn being 0 to 8 mol%.

3. The hydrogen gas detecting element as claimed in Claim 1 and 2, wherein said element body comprises Pd and Sb dispersed on $SnO_2$, Pd/Sn being 0.1 to 8 mol%, Sb/Sn being 0.2 to 5 mol%.

4. The hydrogen gas detecting element as claimed in Claim 1, wherein said element body comprises Pd and Pt, or Pd, Pt and Sb dispersed on $SnO_2$, Pd/Sn being 0.1 to 8 mol%, Pt/Sn being 0.5 to 8 mol% and Sb/Sn being 0 to 8 mol%.

5. The hydrogen gas detecting element. as claimed in Claim 1 and 4, wherein said element body comprises Pd, Pt and Sb dispersed on $SnO_2$, Pd/Sn being 0.1 to 8 mol%, Pt/Sn being 0.5 to 8 mol% and Sb/Sn being 0.2 to 5 mol%.

6. A method of producing a hydrogen gas detecting element which comprises adding $SnO_2$ to a $PdCl_2$ solution to provide a Pd/Sn ratio of 0.1 to 8 mol%, thoroughly dispersing said $SnO_2$ in the solution, drying the mixture, adding antimony oxychloride (SbOCl) to the above dried product to provide a Sb/Sn ratio of 0 to 8 mol%, adding the resulting mixture to an organic solvent to form a paste, coating said paste on an alumina porcelain tube equipped with electrodes, drying said paste to form an element body comprising $SnO_2$ and a $PdCl_2$ or $SnO_2$, $PdCl_2$ and SbOCl-containing layer, sintering said element body in an air atmosphere or an antimony oxide gas atmosphere, providing the sintered element body with a heater, heating the sintered element body to $300 \pm 50°C$ with the heater to age the sintered element body, and treating the sintered element body by heating it at 300 to 350°C in a silane gas atmosphere having a silane content of 500 to 5,000 ppm to disperse a Si oxide on the element body.

7. A method of producing a hydrogen gas detecting element which comprises preparing an aqueous palladium chloride ($PdCl_2$) solution, preparing an aqueous chloroplatinic acid ($H_2PtCl_6$) solution, adding the aqueous palladium chloride solution and the aqueous chloroplatinic acid solution to $SnO_2$ to provide a Pd/Sn ratio of 0.1 to 8 mol% and a Pt/Sn ratio of 0.5 to 8 mol%, thoroughly dispersing $SnO_2$ in the resulting mixture, drying the mixture to form a dried product, adding and mixing antimony oxychloride (SbOCl) to the dried product to provide a Sb/Sn ratio of 0 to 8 mol%, adding the mixture to an organic solvent to form a paste, coating the paste on an alumina porcelain tube equipped with electrodes, drying the paste to form an element body comprising an $SnO_2$, $PdCl_2$ and $H_2PtCl_6$-containing layer or an $SnO_2$, $PdCl_2$, $H_2PtCl_6$ and SbOCl-containing layer, sintering said element body, in an air atmosphere or an antimony oxide gas atmosphere, providing the sintered element body with a heater, heating the sintered element body to $300°C \pm 50°C$ with the heater to age the element body, and treating the sintered element body by heating it at 300 to 350°C in a silane gas atmosphere having a silane content of 500 to 5,000 ppm to disperse a Si oxide on the element body.

8. The method as claimed in Claim 6 or 7, wherein the drying after the $SnO_2$ is thoroughly dispersed is quick freeze drying.

9. The method as claimed in Claim 6 to 8, wherein the treatment of the sintered element body in a silane gas atmosphere is carried out two or more times.

**Revendications**

1. Elément de détection de gaz hydrogène produit par les étapes consistant à disperser de l'oxyde de Si sur un élément en traitant ledit élément dans une atmosphère de gaz silane ayant une teneur en silane de 500 à 5.000 ppm, ledit élément contenant Pd ou Pd et Pt, ou Pd, Pt et Sb dispersés sur $SnO_2$, Pd/Sn étant compris entre 0,1 et 8% en moles, Pt/Sn étant compris entre 0 et 8% en moles, et Sb/Sn étant compris entre 0 et 8% en moles.

2. Elément de détection suivant la revendication 1, caractérisé en ce que l'élément précité comprend Pd ou Pd et Sb dispersés sur $SnO_2$, Pd/Sn étant compris entre 0,1 et 8% en moles, Sb/Sn étant compris entre 0 et 8% en moles.

3.  Elément de détection suivant les revendications 1 et 2, caractérisé en ce que l'élément précité comprend Pd et Sb dispersés sur $SnO_2$, Pd/Sn étant compris entre 0,1 et 8% en moles, Sb/Sn étant compris entre 0,2 et 5% en moles.

4.  Elément de détection suivant la revendication 1, caractérisé en ce que l'élément précité comprend Pd et Pt, ou Pd, Pt et Sb dispersés sur $SnO_2$, Pd/Sn étant compris entre 0,1 et 8% en moles, Pt/Sn étant compris entre 0,5 et 8% en moles et Sb/Sn étant compris entre 0 et 8% en moles.

5.  Elément de détection suivant les revendications 1 et 4, caractérisé en ce que l'élément précité comprend Pd, Pt et Sb dispersés sur $SnO_2$, Pd/Sn étant compris entre 0,1 et 8% en moles, Pt/Sn étant compris entre 0,5 et 8% en moles et Sb/Sn étant compris entre 0,2 et 5%, en moles.

6.  Procédé de production d'un élément de détection de gaz hydrogène qui consiste à ajouter $SnO_2$ à une solution de $PdCl_2$ pour fournir un rapport Pd/Sn de 0,1 à 8% en moles, à disperser complètement ledit $SnO_2$ dans la solution, à sécher le mélange, à ajouter de l'oxychlorure d'antimoine (SbOCl) au produit séché ci-dessus pour fournir un rapport Sb/Sn de 0 à 8% en moles, à ajouter le mélange résultant à un solvant organique pour former une pâte, à revêtir ladite pâte sur un tube de porcelaine alumine équipé d'électrodes, à sécher ladite pâte pour former un élément comprenant $SnO_2$ et une couche contenant $PdCl_2$ ou $SnO_2$, $PdCl_2$ et SbOCl, à fritter ledit élément dans une atmosphère d'air ou dans une atmosphère de gaz oxyde d'antimoine, à munir l'élément fritté d'un système de chauffage, à chauffer l'élément fritté à 300 ± 50°C avec le système de chauffage pour vieillir l'élément fritté, et à traiter l'élément fritté en le chauffant à 300-350°C dans une atmosphère de gaz silane possédant une teneur en silane de 500 à 5.000 ppm pour disperser un oxyde de Si sur l'élément.

7.  Procédé de production d'un élément de détection de gaz hydrogène qui consiste à préparer une solution aqueuse de chlorure de palladium ($PdCl_2$), à préparer une solution aqueuse d'acide chloroplatinique ($H_2PtCl_6$), à ajouter la solution aqueuse de chlorure de palladium et la solution aqueuse d'acide chloroplatinique à $SnO_2$ pour fournir un rapport Pd/Sn de 0,1 à 8% en moles et un rapport Pt/Sn de 0,5 à 8% en moles , à disperser complètement $SnO_2$ dans le mélange résultant, à sécher le mélange pour former un produit séché, à ajouter et mélanger de l'oxychlorure d'antimoine (SbOCl) au produit séché pour fournir un rapport Sb/Sn de 0 à 8% en moles , à ajouter le mélange à un solvant organique pour former une pâte, à revêtir avec cette pâte un tube de porcelaine alumine équipé d'électrodes, à sécher la pâte pour former un élément comprenant une couche contenant $SnO_2$, $PdCl_2$ et $H_2PtCl_6$ ou une couche contenant $SnO_2$, $PdCl_2$, $H_2PtCl_6$ et SbOCl, à fritter ledit élément, dans une atmosphère d'air ou une atmosphère de gaz oxyde d'antimoine, à munir l'élément fritté d'un système de chauffage, à chauffer l'élément fritté à 300°C ± 50°C avec le système de chauffage pour vieillir l'élément, et à traiter l'élément fritté en le chauffant à 300-350°C dans une atmosphère de gaz silane ayant une teneur en silane de 500 à 5.000 ppm pour disperser un oxyde de Si sur l'élément.

8.  Procédé suivant la revendication 6 ou 7, caractérisé en ce que le séchage après que $SnO_2$ soit complètement dispersé est un séchage rapide par congélation.

9.  Procédé suivant les revendications 6 à 8, caractérisé en ce que le traitement de l'élément fritté dans une atmosphère de gaz silane est réalisé deux ou trois fois.

**Patentansprüche**

1.  Ein Wasserstoffgasnachweiselement, hergestellt durch die folgenden Verfahrensschritte: Dispergieren von Si-Oxid auf einem Elementkörper durch Behandeln des genannten Elementkörpers in einer Silangasatmosphäre mit einem Silangehalt von 500 bis 5000 TpM, wobei der genannte Elementkörper Pd oder Pd und Pt, oder Pd, Pt und Sb dispergiert auf $SnO_2$ enthält, das Verhältnis von Pd/Sn 0,1 bis 8 Molprozent, das Verhältnis Pt/Sn 0 bis 8 Molprozent und das Verhältnis Sb/Sn 0 bis 8 Molprozent beträgt.

2.  Das Wasserstoffgasnachweiselement, wie in Anspruch 1 beansprucht, wobei der genannte Elementkörper Pd oder Pd und Sb auf $SnO_2$ dispergiert enthält, wobei das Verhältnis Pd/Sn 0,1 bis 8 Molprozent

und das Verhältnis Sb/Sn 0 bis 8 Molprozent beträgt.

3. Das Wasserstoffgasnachweiselement wie in den Ansprüchen 1 und 2 beansprucht, wobei der genannte Elementkörper Pd und Sb auf $SnO_2$ dispergiert enthält, wobei das Verhältnis von Pd/Sn 0,1 bis 8 Molprozent, und das Verhältnis Sb/Sn 0,2 bis 5 Molprozent beträgt.

4. Das Wasserstoffgasnachweiselement wie in Anspruch 1 beansprucht, wobei der genannte Elementkörper Pd und Pt, oder Pd,Pt und Sb dispergiert auf $SnO_2$ enthält, wobei das Verhältnis Pd/Sn 0,1 bis 8 Molprozent , das Verhältnis Pt/Sn 0,5 bis 8 Molprozent und das Verhältnis Sb/Sn 0 bis 8 Molprozent beträgt.

5. Das Wasserstoffgasnachweiselement wie in den Ansprüchen 1 und 4 beansprucht, wobei der genannte Elementkörper Pd, Pt und Sb auf $SnO_2$ dispergiert enthält, wobei das Verhältnis Pd/Sn 0,1 bis 8 Molprozent, Pt/Sn 0,5 bis 8 Molprozent und Sb/Sn 0,2 bis 5 Molprozent beträgt.

6. Ein Verfahren zur Herstellung eines Wasserstoffgasnachweiselements, welches die Zugabe von $SnO_2$ zu einer $PdCl_2$-Lösung, zwecks Einstellung eines Verhältnisses von Pd/Sn von 0,1 bis 8 Molprozent, das sorgfältige Dispergieren des genannten $SnO_2$ in der Lösung, das Trocknen der Mischung, die Zugabe von Antimonoxychlorid(SbOCl) zu dem genannten getrockneten Produkt unter Einstellung eines Verhältnisses von Sb/Sn von 0 bis 8 Mol-prozent, die Zugabe der resultierenden Mischung zu einem organischem Lösungsmittel zwecks Bildung einer Paste, das Aufbringen der genannten Paste auf ein Aluminiumoxid-Porzellanröhrchen, das mit Elektroden versehen ist, das Trocknen der genannten Paste zwecks Bildung eines Elementkörpers, enthaltend $SnO_2$ und einer $PdCl_2$ oder $SnO_2$. , $PdCl_2$ und SbOCl enthaltenden Schicht, das Sintern des genannten Elementkörpers an Luft oder in einer Antimonoxidgasatmosphäre, das Versehen des gesinterten Elementkörpers mit einem Heizelement, und das Erwärmen des gesinterten Elementkörpers auf eine Temperatur von 300 + 50$^\circ$C mit dem Heizelement zwecks Alterung des gesinterten Elementkörpers und Behandeln des gesinterten Elementkörpers durch Erwärmen desselben auf 300 bis 350$^\circ$C in einer Silangasatmosphäre mit einem Silangehalt von 500 bis 5000 TpM zwecks Dispergieren eines Si-Oxids auf dem Elementkörper umfaßt.

7. Ein Verfahren zur Herstellung eines Wasserstoffgasnachweiselementes, welches das Herstellen einer wäßrigen Palladiumchlorid($PdCl_2$)-Lösung, das Herstellen einer wäßrigen Chlorplatinsäure($H_2PtCl_6$)-Lösung, das Zugeben der wäßrigen Palladiumchloridlösung und der wäßrigen Chlorplatinsäurelösung zu $SnO_2$ zwecks Einstellung eines Pd/Sn-Verhältnisses von 0,1 bis 8 Molprozent und eines Pt/Sn-Verhältnisses von 0,5 bis 8 Molprozent, das sorgfältige Dispergieren von $SnO_2$ in der resultierenden Mischung, das Trocknen der Mischung zwecks Erhalt eines getrockneten Produkts, die Zugabe zu und das Mischen von Antimonoxychlorid(SbOCl) mit dem getrockneten Produkt zwecks Einstellung eines Sb/Sn-Verhältnisses von 0 bis 8 Molprozent, die Zugabe der Mischung zu einem organischen Lösungsmittel zwecks Bildung einer Paste, das Aufbringen der Paste auf ein mit Elektroden versehenes Aluminiumoxid-Porzellanröhrchen, das Trocknen der Paste zwecks Bildung eines Elementkörpers, umfassend eine $SnO_2$, $PtCl_2$ und $H_2PtCl_6$-haltige Schicht oder eine $SnO_2$, $PdCl_2$, $H_2PtCl_6$ und SbOCl-haltige Schicht, das Sintern des genannten Elementkörpers an Luft oder in einer Antimonoxidgasatmosphäre, das Versehen des gesinterten Elementkörpers mit einem Heizelement, das Erwärmen des gesinterten Elementkörpers auf 300$^\circ$C + 50$^\circ$C mittels des Heizelementes zwecks Alterung des Elementkörpers und das Behandeln des gesinterten Elementkörpers durch Erwärmen desselben auf 300 bis 350$^\circ$C in einer Silangasatmosphäre mit einem Silangehalt von 500 bis 5000 TpM zwecks Dispergieren eines Si-Oxids auf dem Elementkörper, umfaßt.

8. Das Verfahren wie in Anspruch 6 oder 7 beansprucht, in welchem das Trocknen, das nach dem sorgfältigen Dispergieren des $SnO_2$ erfolgt, ein Schnellgefriertrocknen ist.

9. Das Verfahren wie in den Ansprüchen 6 bis 8 beansprucht, in welchem das Behandeln des gesinterten Elementkörpers in einer Silangasatmosphäre zweimal oder mehrmals durchgeführt wird.

Fig. 1

Fig. 2

Fig. 3

Fig. 4

Fig. 5